# EUROPEAN PATENT APPLICATION

(11) **EP 4 261 200 A1**
(43) Date of publication of application: **18.10.2023**
(21) Application number: 20964760.1
(22) Date of filing: 11.12.2020
(51) Int. Cl.: C07C 1/12, B01J 23/00, B01J 23/38, B01J 23/16, B01J 23/70

(54) **METHOD FOR PRODUCING HYDROCARBON MOLECULE BY MEANS OF ENERGY RADIATION**

(71) Applicant: Beijing Guanghe New Energy Technology Co., Ltd., Beijing 100095 (CN)
(72) Inventor: WANG, Cong, Beijing 100095 (CN); HUO, Haibin, Beijing 100095 (CN)
(74) Representative: Engelhard, Markus
(86) International application number: PCT/CN2020/135863
(87) International publication number: WO 2022/120824

(57) **Abstract**

A method for producing a hydrocarbon molecule by means of energy radiation, comprising: contacting a composite catalyst with at least one hydrogen-containing source and at least one carbon-containing source, and radiating energy to the composite catalyst, the hydrogen-containing source, and the carbon-containing source to produce a hydrocarbon molecule, wherein the composite catalyst contains at least one nano-base structure and at least one atom site, and the atom site comprises one or more chemical elements of Mn, Co, Fe, Ru, Rh, Al, Ag, Au, Pt, Pd, Cu, Ni, Zn, Ti, Os, Ir, and La.

## Description

### FIELD OF THE INVENTION

The present invention relates to a method for catalytically producing hydrocarbon molecules by means of energy radiation, which belongs to the field of utilization of carbon dioxide and renewable energy.

### BACKGROUND

The greenhouse effect caused by massively increased carbon dioxide is a great threat to environment, climate and ecology of all humanity. Capture, utilization and sequestration technologies of carbon dioxide are getting more and more attentions, and scientists around the world have conducted a large number of research projects around these technologies. Artificial photosynthesis technology is one of the most promising technologies, which converts carbon dioxide into organic compound forms such as hydrocarbons, alcohols and the like by utilizing solar energy, so as to achieve sequestration and utilization of carbon dioxide, and energy recycling.

During the past decades, the field of converting solar energy into more useful energy sources has attracted increasing interests. Some technologies have shown great hope in this field, but there is still a long way to go before commercialization. So far, most research works can only successfully synthesize short chain (C₁-C₂) hydrocarbon molecules, and their conversion efficiencies for converting solar energy to chemical energy were 1 or 2 orders of magnitude lower than that of natural photosynthesis. A few works could synthesize long chain hydrocarbon molecules (>C₃, or even >Cs), but current catalyst stability still cannot provide reliable support for industrialization. Therefore, the most important task currently is to develop more efficient and stable catalysts.

Plasmonic catalysts could immensely enhance local energy on surfaces of a nanostructure due to plasmon effect. Accordingly, in a case of the overall reaction conditions are mild, the catalytic reaction could be efficiently promoted such that it is possible to conduct a reaction that could not be conducted under normal temperature and normal pressure.

The concept of single atom catalysis received widespread concern and research since it was proposed in recent years. As development of advanced characteristic technology, single atom catalysts provide the possibility to explain the structure-activity relationship of catalysts from molecular and atomic level, and to connect heterogeneous catalysis with homogeneous catalysis. Single atom catalysts show different activities, selectivities and stabilities from conventional nano catalysts, due to its special structures.

Therefore, it is possible to develop catalysts for artificial photosynthesis with efficiencies and stabilities that satisfy commercial requirements, by coupling advantages of both plasmon effect with single atom catalysis.

### SUMMARY OF THE INVENTION

On the basis of the technical problems exist in the technical background, the present invention provides a novel plasmonic catalytic technology, the plasmonic catalyst comprising atomic sites such as single atomic sites and/or atomic clusters having 2-25 atoms, and provides a special method for preparing hydrocarbons molecules from CO or CO₂ in industrial flue gas or atmosphere by means of light radiation and/or heat radiation, in the presence of a cost-effective catalyst.

One aspect of the present invention is a method for producing hydrocarbon molecules by means of energy radiation, comprising:
contacting a composite catalyst with at least one hydrogen-containing source and at least one carbon-containing source, and
subjecting the composite catalyst, the hydrogen-containing source and the carbon-containing source to energy radiation, to produce hydrocarbon molecules, wherein
the composite catalyst comprises at least one nano-base structure and at least one atomic sites, the atomic sites comprise one or two or more chemical elements selected from Mn, Co, Fe, Ru, Rh, Al, Ag, Au, Pt, Pd, Cu, Ni, Zn, Ti, Os, Ir and La.

In certain embodiments, the energy radiation is at least on selected from light radiation and heat radiation, preferably light radiation.

In certain embodiments, a distance between the nano-base structure and the atomic sites is 5 nm or less, preferably 1 nm or less, more preferably less than 0.1 nm, and most preferably the nano-base structure and the atomic site are in close contact with each other.

In certain embodiments, the atomic sites are bonded with the nano-base structure, for example by physical manner or chemical manner.

In certain embodiments, a mass percentage of the atomic sites and the nano-base structure is 50% or less, preferably 0.01% to 30%, preferably 0.01% to 5%, more preferably 0.1% to 2%, most preferably 0.1% to 1%.

In certain embodiments, the atomic sites are loaded on surface, internal pores of the nano-base structure, or distributed in internal crystal lattices of the nano-base structure, preferably respective atomic sites are dispersed uniformly. In preferred embodiments, intervals between respective atomic sites are 0.2-500nm, preferably 1-50nm, more preferably 1-10nm.

In preferred embodiments, the nano-base structure is selected from a group consisting of Mn, Co, Ce, Fe, Al, Ca, Ce, Cu, Ni, Ti, Zn, Si, Mo, Bi, V, C, N and oxides, nitrides, sulfites, carbides, hydroxides, chlorides thereof and metal-organic frameworks (MOF), preferably metal-organic frameworks, TiO₂ or Al₂O₃.

In certain embodiments, the composite catalyst is a catalyst in which Co and Mn are loaded on or bonded to a metal-organic framework (CoMn-MOF), a catalyst in which Fe is loaded on or bonded to Al₂O₃ (Fe-Al₂O₃), a catalyst in which Co is loaded on or bonded to Al₂O₃ (Co-Al₂O₃), a catalyst in which Ru is loaded on or bonded to Al₂O₃ (Ru-Al₂O₃), a catalyst in which Ru and Fe are loaded on or bonded to Al₂O₃ (RuFe-Al₂O₃), or a catalyst in which Ru and Co are loaded on or bonded to Al₂O₃ (RuCo- Al₂O₃).

In preferable embodiments, the nano-base structure is about 1 nm to about 1000 nm, preferably about 70 nm to about 1000 nm, about 100 nm to about 800 nm, about 200 nm to 500 nm in at least one dimension of length, width and height.

In preferable embodiments, the nano-base structure each independently is about 1 nm to about 3000 nm in length, width or height, preferably about 100 nm to about 3000 nm, about 500 nm to about 2500 nm, or about 1000 nm to about 2000 nm in length, and/or about 1 nm to about 1000 nm, about 70 nm to about 1000 nm, about 100 nm to about 800 nm, or about 200 nm to about 500 nm in width or height, or
the nano-base structure each independently has an aspect ratio of about 1 to about 20, preferably an aspect ratio of about 1 to about 10, or about 2 to about 8.

In certain embodiments, the nano-base structure each independently has a shape of spherical, spike, flake, needle, blade of grass, columnar, polyhedral, 3D cone, cuboidal, sheet, hemispherical, irregular 3D shape, porous structure or any combinations thereof.

In certain embodiments, a plurality of the atomic sites are arranged in a patterned configuration, preferably in a plurality of layers, on the nano-base structure, or
a plurality of the atomic sites are dispersed randomly in the nano-base structure and/or on an surface of the nano-base structure.

In certain embodiments, the energy radiation allows the reaction progresses at a temperature between about 20°C to about 500°C, preferably about 50°C to about 300°C, about 70°C to about 250°C, about 90°C to about 200°C, about 100°C to about 200°C, about 100°C to about 180°C, about 100°C to about 150°C, about 110°C to about 140°C, about 120°C to about 140°C, and
a catalyst activity per unit for producing hydrocarbon molecules is more than 18 µmol g⁻¹ h⁻¹, and more than 50 µmol g⁻¹ h⁻¹ in preferred temperature ranges.

In certain embodiments, the reaction is initiated by utilizing light radiation or heat radiation, and the reaction is continued to progress by utilizing light radiation or heat radiation, wherein
a power of the light radiation is 200-1500 W/m², preferably 200-1000 W/m², and most preferably 500-1000 W/m².

In certain embodiments, temperatures of the composite catalyst, the hydrogen-containing source and the carbon-containing source are raised by the light radiation, preferably the light radiation is the sole source for raising the temperatures.

In certain embodiments, the carbon-containing source is selected from a group consisting of CO₂, CO, C₁₋₄ hydrocarbons, synthesis gas, bicarbonate salts and any combination thereof, or air, industrial flue gas, exhausts or emissions comprising one or more of these carbon-containing sources, preferably CO₂ or CO.

In certain embodiments, the hydrogen-containing source is selected from a group consisting of water, H₂, C₁₋₄ hydrocarbons and any combination thereof, or air, industrial flue gas, exhausts or emissions comprising one or more of these hydrogen-containing sources, preferably water.

In certain embodiments, when the atomic sites includes two or more chemical elements and are single atoms, the two or more elements may be arranged at intervals, or may be arranged randomly.

In certain embodiments, when the atomic sites are atomic clusters, compositions of respective atomic clusters may be same or different, for example, respective atomic clusters may include different elementary composition, and/or include different numbers of atoms.

### BRIEF DESCRIPTION OF THE DRAWINGS

Fig. 1 illustrates a transmission electron microscopy (TEM) image of a Co-Al₂O₃ composite catalyst.
Fig. 2 illustrates a transmission electron microscopy (TEM) image of a Fe-Al₂O₃ composite catalyst.
Fig. 3 illustrates stability of a CoMn-MOF plasmonic composite catalyst for producing hydrocarbons.
Fig. 4 illustrates catalyst activities per unit of Co-Al₂O₃ plasmonic composite catalyst having different loading amounts and Co nanostructure catalyst having different particle diameters for a reaction producing hydrocarbon molecules.

### EMBODIMENTS

The invention demonstrated, unexpectedly, that CO₂ or CO and water were converted to hydrocarbon molecules, using light radiation and/or heat radiation as energy input, in the presence of composite catalysts having plasmon effect.

Before further description of the present invention, certain terms employed in the specification, examples and appended claims are defined in the following section. The definitions listed herein should be read in light of the remainder of the disclosure and understood as by those skilled in the art. Unless defined otherwise, all technical and scientific terms as used herein have the same meaning as commonly understood by a person of ordinary skill in the art to which this invention belongs

### DEFINITIONS

The term "catalyst" as used herein refers to substances that exhibit effects to increase the rate of a chemical reaction by reducing the activation energy of the reaction. The rate increasing effect is referred as "catalysis". Catalysts are not consumed in a catalytic reaction, therefore they can continue to catalyze the reaction of further quantities of reactant with a small amount.

The term "plasmon provider" as used herein refers to conductor whose real part of dielectric constant thereof is negative. Plasmon provider provides surface plasmon when excited by electromagnetic radiation.

The term "temperature-dependence" as used herein refers to properties that may vary when temperature changes by a given level. The temperature difference to alter the property may be any degree, such as 0.1°C, 1°C, 5°C, 10°C, 100°C, or 1000°C.

The term "chemical element" as used herein refers to chemical substance consisting of atoms having the same number of protons in their atomic nuclei. Specifically, chemical elements are those recorded in the periodic table of chemical elements. Chemical elements include natural elements and synthetic elements. Chemical elements also include elements not discovered yet with more than 118 protons in the atomic nuclei.

The term "bond" or "load" as used herein refers to bonded to or loaded on the surface, in the internal holes or internal lattices by physical manner or chemical manner, wherein the physical manner includes Van der Waals force, metallic bond and other conventional physical bonding manner, and the chemical manner includes ionic bond, covalent bond, coordination bond and other conventional chemical bonding manner.

The term "alloy" as used herein refers to a mixture of metals or a mixture of metal and other elements. Alloys are defined by metallic bonding character. An alloy may be a solid solution of metal element (a single phase) or a mixture of metallic phases (two or more phases).

The term "metal-organic frameworks (MOF)" as used herein refers to organic-inorganic hybrid materials having intramolecular pores or metal-organic skeleton structure having periodic network structures formed from organic ligands and metal ions or clusters by means of self-assembly. The MOF may contain transition metals, rare-earth metals, main group metals such as alkali metals and alkaline-earth metals, for example, Cu, Zn, Cd, Fe, Ti, Mn, Al and Co, preferably Ti may be contained, non-metallic elements such as O, N, S, P, halogen (such as F, Cl, Br, I) and the like may be further contained. The MOF may be prepared by known methods in the art such as evaporation solvent method, diffusion method, hydrothermal or solvothermal method, ultrasonic and microwave method and the like.

The term "catalyst activity per unit" as used herein refers to a molar quantity of a product produced in unit time by active catalyst per unit mass, under certain reaction conditions. Specifically, catalyst activity per unit = a molar quantity of a reaction product/a mass of active catalyst/reaction time.

The term "close contact" as used herein refers to that there is substantially no gap between the two components, e.g. a distance between the two components is 1 nm or less, 0.1 nm or less, or substantially 0 nm, preferably metallic bonds or coordinate bonds are formed between the two components.

The term "C₁₋₄ hydrocarbons" as used herein includes C₁, C₂, C₃ and C₄ hydrocarbons, such as methane, ethane, n-propane, isopropane, n-butane, and isobutane.

### Plasmonic composite catalyst

One aspect of the present invention is a plasmonic composite catalyst for producing hydrocarbon molecules by light radiation and/or heat radiation.

Without wishing to be bound by theory, the plasmonic composite catalyst of the present invention interacts with the raw materials of the reaction to reduce the activation energy of the reaction, so as to initiate the reaction by utilizing light radiation and/or heat radiation, and to increase the reaction rate.

The plasmonic composite catalyst includes two structures: atomic sites and nano-base structure, wherein the atomic sites and the nano-base structure are contact with each other. In preferred embodiments, in the plasmonic composite catalyst, a mass percentage of the atomic sites and the nano-base structure is 50% or less, preferably 0.01% to 30%, preferably 0.01% to 5%, more preferably 0.01% to 2%, most preferably 0.1% to 1%.

### Atomic sites

The term "atomic sites" as used herein refers to mutually independent metal single atoms and/or mutually independent atomic clusters including 2-25, preferably 2-20 metal atoms, the metal single atoms and/or the atomic clusters are stably bonded to or loaded on surface and/or in internal pores and/or internal crystal lattices of the nano-base structure, preferably uniformly dispersed on the nano-base structure, more preferably uniformly dispersed on the surface of the nano-base structure. The metal single atoms or atoms in the atomic clusters are present in a valance state between zero valance state and the highest valance state that the metal usually present, an average valance state of the metal is, for example, 0 to +4, or 0 to +3, or 0 to +2, or 0 to +1, preferably 0. The distances between atoms in the atomic clusters are less than 1nm, preferably 0.1-0.5 nm.

Atoms in the atomic sites are bonded to atoms in the nano-base structure by physical manner or chemical manner, for example coupling by Van der Waals force, metallic bond and other conventional physical bonding manner, or bonding by ionic bond, covalent bond, coordination bond and other conventional chemical bonding manner, or forming complex by coordinate bonds.

When the atomic sites are mutually independent metal single atoms, the interaction between the metal single atoms and the atoms in the nano-base structure may prevent the metal single atoms from being agglomerated, and thus make it more stable. In certain embodiments, in the catalytic metal single atomic sites, all of the catalytic metal are present in form of isolated atoms, that is, a degree of dispersion of the catalytic metal atoms is 100%, and thus the catalytic metal atoms can be utilized to the maximum; preferably, all of the catalytic metal atoms are directly fixed to the surface of the nano-base structure and become 100% interface atoms, and thus the interaction of metal-base interface can be fully utilized to optimize the catalytic performance.

When the atomic sites are mutually independent metal atomic clusters, the metal atomic clusters are bonded with atoms in the nano-base structure by physical manner or chemical manner. The metal atomic clusters are dispersed on the nano-base structure and/or in the nano-base structure.

When the atomic sites are single atoms of single metal element, in certain embodiments, the single metal element works as both plasmon provider and catalytic property provider, the nano-base structure provides physical supporting function; in other embodiments, the single metal element works as plasmon provider, and the nano-base structure provides physical supporting function and works as catalytic property provider.

When the atomic sites are metal atomic clusters, in some embodiments, some atomic clusters comprising specific elements work as plasmon provider, other atomic clusters comprising specific elements work as catalytic property provider, the nano-base structure provides physical supporting function; in other embodiments, the atomic clusters work as plasmon provider, and the nano-base structure provides physical supporting function and works as catalytic property provider.

In other embodiments, the atomic sits and the nano-base structure work together as both plasmon provider and catalytic property provider, and the nano-base structure provides physical supporting function.

### NANO-BASE STRUCTURE

The term "nano-base structure" as used herein refers to a structure having a range of nanometer-scale, i.e. about 1 nm to about 1000 nm, preferably about 70 nm to about 1000 nm, about100 nm to about 800 nm, about 200 nm to about 500 nm in at least one dimension of length, width, and height. The nano-base structure can have one dimension which exceeds 1000 nm, for example, have a length in a range of micrometer-scale, such as 1 µm to 5 µm. In certain cases, tubes and fibers with only two dimensions within a range of nanometer-scale are also considered as the nano-base structures. Material having nano-base structure may exhibit size-related properties that differ significantly from those observed in bulk materials.

The nano-base structure of the present invention each independently is about 1 nm to about 3000 nm in length, width or height. The length thereof is preferably about 100 nm to about 3000 nm, more preferably about 500 nm to about 2500 nm, further more preferably about 1000 nm to about 2000 nm. The width or height thereof is preferably about 1 nm to about 1000 nm, preferably about 70 nm to about 1000 nm, more preferably about 100 nm to about 800 nm, further more preferably about 200 nm to about 500 nm.

The nano-base structure of the present invention each independently has an aspect ratio (i.e., a ratio of length to width/height) of about 1 to about 20, preferably an aspect ratio of about 1 to about 10, or about 2 to about 8. The nano-base structure of the present invention can also have a relatively small aspect ratio such as about 1 to about 2.

The nano-base structure of the present invention each independently has a shape of spherical, spike, flake, needle, blade of grass, columnar, polyhedral, 3D cone, cuboidal, sheet, hemispherical, irregular 3D shape, porous structure or any combinations thereof.

The nano-base structure is selected from a group consisting of Mn, Co, Ce, Fe, Al, Ca, Ce, Cu, Ni, Ti, Zn, Si, Mo, Bi, V, C, N and oxides, nitrides, sulfites, carbides, hydrocarbon molecules oxides, chlorides thereof and metal-organic frameworks.

The term "nano-base structure" as used herein comprises more than 25, preferably more than 30 atoms.

A plurality of the nano-base structures of the present invention can be arranged in a patterned configuration, preferably in a plurality of layers, or a plurality of the nano-base structures may be randomly dispersed in a medium. For example, the nano-base structures may be bound to a substrate. In such case, the nano-base structures are generally not aggregated together, but rather, are arranged or stacked in an orderly pattern. Alternatively, a plurality of the nano-base structures can be dispersed in a liquid medium, in which each nano-base structure is free to move with respect to any other nano-base structure.

For example, the nano-base structure could have a spike or blade of grass-like geometric configuration. Optionally, the nano-base structure has a flaky geometric configuration with a relative thin thickness. Preferably, the nano-base structure has a configuration of nano-jungle, nano-grass, and/or nano-snowflake. The nano-base structure could have a relative large aspect ratio, such nanostructure could have a construction of nano-spike, nano-snowflake or nano-needle. The aspect ratio could be about 1 to about 20, about 1 to about 10, or about 2 to about 8. Preferably, the length of the nano-base structure could be about 100 nm to about 3000 nm, about 500 nm to about 2500 nm, or about 1000 nm to about 2000 nm; the width or the height could be about 1nm to about 1000 nm, about 70 nm to about 1000 nm, about 100 nm to about 800 nm, or about 200 nm to about 500 nm.

The nano-base structures may be bound to a substrate. Accordingly, the nano-base structures are generally not aggregated together, but rather, are arranged in an orderly pattern. The substrate can be formed of metal or polymer material (e.g., polyimide, PTFE, polyester, polyethylene, polypropylene, polystyrene, polyacrylonitrile, etc.)
In other embodiments, the nano-base structure has a shape of spherical, columnar, polyhedral, 3D cone, cuboidal, sheet, hemispherical, irregular 3D shape, porous structure or any combinations thereof. Such nano-base structures each independently is about 1 nm to about 1000 nm, preferably about 70 nm to about 1000 nm, about 100 nm to about 800 nm, or about 200 nm to about 500 nm in length, width or height.

Furthermore, the plasmonic atomic catalyst of the present invention could function in various states, such as dispersed, congregated, or attached/grew on surface of other materials. In preferred embodiments, the plasmonic atomic catalyst are dispersed in a medium, the medium is preferably the reactants of the reaction, such as water.

### METHOD FOR PRODUCING HYDROCARBON MOLECULES

Another aspect of the present invention is a method for producing hydrocarbon molecules by light radiation and/or heat radiation, comprising:
contacting one plasmonic composite catalyst with at least one hydrogen-containing source and at least one carbon-containing source; and
subjecting the plasmonic composite catalyst, the hydrogen-containing source and the carbon-containing source to light radiating and/or heat radiating, to produce hydrocarbon molecules.

Under catalytic effects of the plasmonic composite catalyst, the energy radiation, i.e., light radiation and/or heat radiation, initiates the reaction for synthesizing hydrocarbon molecules. Without wishing to be bound by theory, the plasmonic composite catalyst could convert and transmit energy of the light radiation and the heat radiation, so as to keep the reaction of the present invention progressing. In certain temperature ranges, raising the temperature leads to a higher energy conversion rate of producing hydrocarbon molecules.

The light radiation and/or heat radiation step is carried out at a temperature between about 20°C to about 800°C, about 20°C to about 500°C, about 50°C to about 300°C, about 70°C to about 250°C, about 90°C to about 200°C, about 100°C to about 200°C, about 100°C to about 180°C, about 100°C to about 150°C, about 110°C to about 140°C, and about 120°C to about 140°C. A catalyst activity per unit for producing hydrocarbon molecules is more than 18 µmol g⁻¹ h⁻¹ in the above temperature ranges, and more than 50 µmol g⁻¹ h⁻¹ in preferred temperature ranges.

The term "heat" as used herein refers to thermal energy transferred from one system to another as a result of thermal interactions. Heat may be transferred into the reaction system from an external heat source, alternatively, heat may be carried by one component of the reaction so as to be transferred to other components involved in the reaction. In other words, the component that carries heat before reaction is an internal heat source. In certain embodiments, temperatures of the plasmonic composite catalyst, the hydrogen-containing source and the carbon-containing source during the reaction of the present invention is raised by heat radiation.

In the reaction of the present invention, light radiation mimics the wavelength composition and strength of solar light, thus the temperature of the radiated catalyst and reactants mixture could be raised. When the radiation intensity reaches a certain level, the temperatures of the plasmonic composite catalyst, the hydrogen-containing source and carbon-containing source are raised by the light radiation. Preferably, light radiation is the sole source for raising the temperature.

In the reaction of the present invention, after the reaction is initiated, the reaction is continued to progress under light radiation. The term "light" as used herein refers to electromagnetic wave having a wavelength from about 250 nm to about 2000 nm. In other words, light refers to the radiance of visible light. Preferably, in the reaction of the present invention, a power of light radiation is less than the power of solar radiation (i.e., the solar constant). For example, the power of light radiation is 200-1500 W/m², preferably 200-1000 W/m², and most preferably 500-1000 W/m². The light radiation could be solar light or light emitted from artificial light sources, wavelength of the light radiation is between about 250 nm to about 2000 nm.

The reaction time varies depending on reaction scale, radiation intensity, temperature and other factors, the reaction is continuously performed utilizing a well-established apparatus with a continuous feed of the hydrogen-containing and the carbon-containing source. The reaction time could be 0.1 hour or more, preferably 0.1 hour to 1000 hours, preferably 0.1 hour to 500 hours, preferably 0.5 hour to 100 hours, preferably 1 hour to 50 hours, preferably 2 hours to 30 hours, most preferably 4 hours to 20 hours.

The reaction could be carried out at low pressure, normal pressure or high pressure, the reaction pressure could be properly selected based on reaction scale, radiation intensity, temperature and other factors, for example, the reaction pressure can be at least 1 bar, e.g. 1 bar to 30 bar, preferably 1 bar to 20 bar, more preferably 1.5 bar to 5 bar.

### REACTION MATERIALS

The carbon-containing source is selected from a group consisting of CO₂, CO, C₁₋₄ hydrocarbons, synthesis gas, bicarbonate salts and any combination thereof, or air, industrial flue gas, exhausts or emissions comprising one or more of these carbon-containing sources, preferably CO₂ or CO.

The hydrogen-containing source is selected from a group consisting of water, H₂, C₁₋₄ hydrocarbons and any combination thereof, or air, industrial flue gas, exhausts or emissions comprising one or more of these hydrogen-containing sources, and preferably water.

As can be seen from the above reaction materials, the carbon-containing source and the hydrogen-containing source that could be utilized in the present invention are contained widely in industrial exhaust gas, waste water, flue gas, combustion emission and automobile exhaust, which could be used as the reaction materials of the present invention, so as to promote recycling of industrial wastes.

### REACTION PRODUCTS

Hydrocarbon molecules can be produced by the reaction of the present invention. Without wishing to be bound by theory, decompositions and recombinations of various reaction material molecules on the atomic sites and the nano-base structure of the plasmonic composite catalysts could be included in the reaction mechanism of the present invention.

### EXAMPLES

### Example 1 Preparation of the plasmonic composite catalyst

### Preparation method of Co-Al₂O₃ (Co was loaded on or bonded to Al₂O₃) composite catalyst:

3.0 g of nano active alumina (Al₂O₃, γ-crystal form, 10-20nm), 2.7 g of lithium chloride (LiCl), 3.3 g of potassium chloride (KCl) and 60 mg of cobalt chloride hexahydrate (CoCl₂·6H₂O) were added into an agate mortar and ground for 30 min. The ground powder was spread on bottom of a quartz boat and then placed in a tube furnace. The tube furnace was purged with nitrogen (N₂) with a flow rate of 80 ml/min for 30 minutes at first, then heated to 500°C with a heating rate of 8°C/min without changing the flow rate of nitrogen. After calcined for 4 hours, the tube furnace was cooled down to room temperature. The calcined sample was placed into a 500 mL beaker, and then immersed with 500 mL deionized water for about 4 hours. After removing the supernate after immersion, the solution was washed with deionized water for 6 times and centrifugally separated at 8000 rpm, then dried at 80°C for 12 h in an oven, to obtain the Co-Al₂O₃ composite catalyst having a loading amount of 0.5% used in experiments.

Co-Al₂O₃ composite catalysts having loading amounts of 0.25% and 1%, respectively, were obtained by adjusting additive amounts of cobalt chloride hexahydrate.

Preparation methods of Fe-Al₂O₃ (Fe was loaded on or bonded to Al₂O₃), Ru-Al₂O₃ (Ru was loaded on or bonded to Al₂O₃), RuFe-Al₂O₃ (Ru and Fe were loaded or bonded to Al₂O₃), and RuCo-Al₂O₃ (Ru and Co were loaded on or bonded to Al₂O₃):
(1) 0.125 g of ferric acetylacetonate and 50 g of aluminum acetylacetonate were put into a 100ml zirconia ball milling tank evenly.
(2) 50 zirconia balls having a diameter of 6 mm and 20 zirconia balls having a diameter of 10 mm were put into the tank, and ball milled with a planetary ball mill for 10 h at a rotation rate of 400 rpm/min.
(3) The powder was taken out and placed in a porcelain boat and calcined in air atmosphere in a muffle furnace at 400°C for 5 h.
(4) The Fe-Al₂O₃ composite catalyst having a loading amount of 0.25% was obtained by taking out the calcined product.
(5) The Ru-Al₂O₃ composite catalyst having a loading amount of 0.25% was obtained by replacing 0.125 g of ferric acetylacetonate in the above preparation procedure with 0.125 g of ruthenium acetylacetonate. RuFe-Al₂O₃ composite catalyst having Ru loading amount of 0.125% and Fe loading amount of 0.125%, and RuCo-Al₂O₃ composite catalyst having Ru loading amount of 0.125% and Co loading amount of 0.125% were obtained by replacing 0.125 g of ferric acetylacetonate in the above preparation procedure with 0.0625 g of ruthenium acetylacetonate and 0.0625 g of ferric acetylacetonate, or 0.0625 g of ruthenium acetylacetonate and 0.0625 g of cobalt acetylacetonate, respectively.

CoMn-MOF (Co and Mn were loaded on or bonded to MOF) composite catalyst was prepared according following method:

### 1. Preparation of organic-metal frameworks (MOF) base

4.2 mL of isopropyl titanate and 7.06g of terephthalic acid were added to a mixed solution containing 108 mL of N,N-dimethylformamide and 12 mL of methanol. The mixture was magnetically stirred for 30 min at a temperature of 25°C, until a transparent and uniform solution was obtained. The solution was transferred into a 200 mL stainless steel high-pressure reactor, then the reactor was put in an oven and heated to 150°C to conduct a reaction for 16 hours. After cooling down to the room temperature, three methanol washes, centrifugation, and drying at 80°C for 16 hours in an oven, a MOF base was obtained and reserved. After characterized by SEM, the MOF base showed morphology of columnar nanoparticles having a diameter of 700nm and a height of 200nm.

### 2. Preparation of CoMn-MOF catalyst

2 g of the MOF base and 100 mL of methanol were added into a 250 mL beaker and magnetically stirred evenly. 18 mg of manganese nitrate tetrahydrate (Mn(NO₃)₂•4H₂O) and 12 mg of cobalt nitrate hexahydrate (Co(NO₃)₂•6H₂O) were weighed in a 100 mL beaker, 20 mL of methanol was added and then stirred until dissolved. The mixed liquid was added to the MOF dispersion. The CoMn-MOF catalyst having a Co loading amount of 0.5% and a Mn loading amount of 0.5% was obtained after stirring for 1 h, washing by methanol, filtrating and separating with a Buchner funnel, and drying at 80°C for 16 hours in an oven.

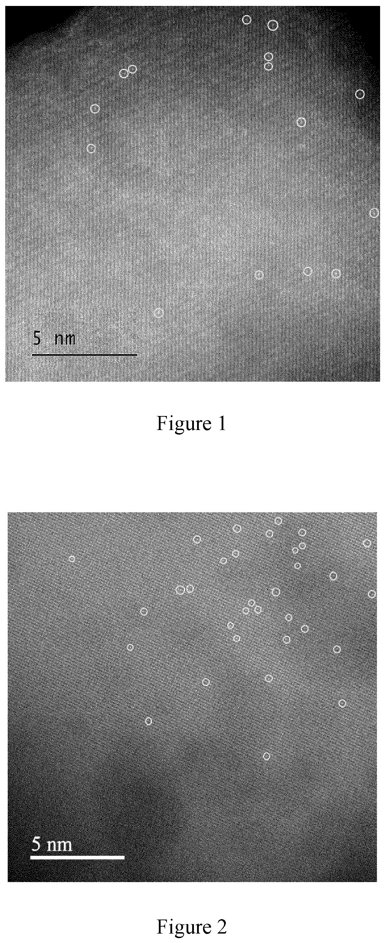

Figs. 1 and 2 showed transmission electron microscopy (TEM) images of the CoAl₂O₃ and Fe-Al₂O₃ plasmonic composite catalyst, in which the atomic sites prepared on the nano-base was labeled with yellow circles (only partially labeled). It can be seen that the atomic sites were relatively evenly dispersed on the base, and intervals between the atomic sites were about 1-10 nm.

### Example 2 Photocatalytic reaction for producing hydrocarbon molecules

The CoMn-MOF composite catalyst prepared in Example 1 was used, in which the MOF (metal-organic framework) mainly containing Ti metal element functioned as the nano-base structure. After characterized by SEM, the MOF base was columnar nanoparticles having a diameter of 700 nm and a height of 200 nm. The atomic sites including Co and Mn were in a form of atomic clusters formed of 2-4 metal atoms and were dispersed on the surface of the MOF, and intervals between the atomic clusters were 5-10 nm.

1.5 g of CoMn-MOF composite catalyst and 2 mL of ultrapure water were added into a sealable pressure-bearing glass tube having a volume of 35 mL, 4 bar CO₂ was filled into the glass tube after air in the tube was replaced with CO₂. The glass tube was laid flat on glass wool, so that the catalyst and water were tiled evenly, and a halogen lamp with controlled voltage was employed to radiate vertically from above. The intensity of the incident light was about 1000-1500 W/m². Thermocouples were connected to the lower part of the glass tube to monitor the temperature. The temperature of the glass tube was controlled to 135°C±5°C, the radiation was continued for 18 h to conduct the photocatalytic reaction. After reaction, an outlet of the reaction tube was connected to a gas chromatography with a flame ionization detector (GC-FID) and a gas chromatography with a thermal conductivity detector (GC-TCD), to conduct product analysis.

By analysis, a concentration of C₁-C₃ hydrocarbon products in the product obtained from stable reaction was about 1637.5 ppm, and a calculated catalyst activity per unit corresponding to active single atom of the catalyst was about 64.98 µmol/g/h.

### Example 3 Stability of the plasmonic composite catalyst for reaction of producing hydrocarbons

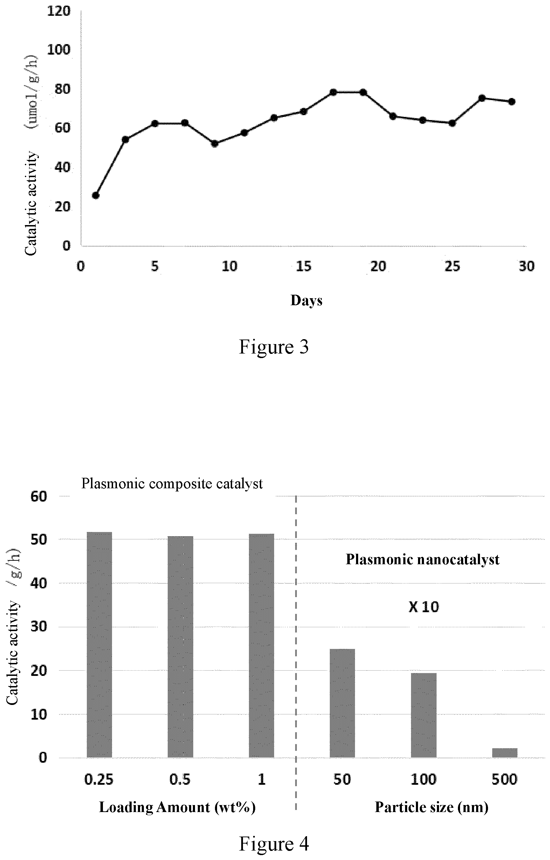

The photocatalytic reaction was continuously carried out for 30 days in the same manner as Example 2 by utilizing the same CoMn-MOF composite catalyst used in Example 2, and the products were analyzed every day. As shown in Fig. 3, the catalyst activity per unit was stabilized at about 60-80 µmol/g/h, which means the reaction for producing hydrocarbon molecules has good stability.

### Example 4 Photocatalytic reaction for producing hydrocarbon molecules

The catalyst was the Fe-Al₂O₃ composite catalyst with Al₂O₃ as the nano-base structure prepared according to Example 1, and showed morphology of irregular spherical particles having diameters of 500-1000 nm after characterized by SEM. Isolated Fe metal single atoms were distributed on the surface and in the pores of Al₂O₃, and intervals between the metal atoms were 1-5 nm.

A photocatalystic reaction was carried out by utilizing 1.5 g of the Fe-Al₂O₃ composite catalyst in the same manner of Example 2.

By analysis, a concentration of C₁-C₃ hydrocarbon products in the product obtained from stable reaction was about 837.6 ppm, and a calculated catalyst activity per unit corresponding to active single atom of the catalyst was about 66.48 µmol/g/h.

### Example 5 Temperature-dependence of the catalytic reaction for producing hydrocarbon molecules

Except the reaction temperature, the photocatalytic reaction was carried out in the same manner as Example 4 by utilizing the same Fe-Al₂O₃ composite catalyst used in Example 4, and the catalyst activity per unit for producing C₁-C₃ hydrocarbon molecules was calculated. The results were shown in following table:

| | Radiation intensity (W/m²) | Reaction temperature (°C) | Reaction time (h) | Catalyst activity per unit (µmol/g/h) |
|---|---|---|---|---|
| 1 | 1000 | 60 | 18 | 0.16 |
| 2 | 1000 | 75 | 18 | 2.54 |
| 3 | 1000 | 90 | 18 | 18.97 |
| 4 | 1000 | 105 | 18 | 41.74 |
| 5 | 1000 | 135 | 18 | 66.48 |
| 6 | 1000 | 150 | 18 | 63.52 |
| 7 | 1000 | 180 | 18 | 51.49 |
| 8 | 1000 | 200 | 18 | 31.63 |

As can be seen from the above experimental results, in a range of 60 to 135°C, the energy conversion rate for producing hydrocarbon molecules in the photocatalytic reaction increased as the temperature raised. When the temperature exceeded 135°C, the energy conversion rate for producing hydrocarbon molecules decreased.

### Example 6 Thermal catalytic reaction for producing hydrocarbon molecules

The catalyst was the Co-Al₂O₃ composite catalyst with Al₂O₃ as the nano-base structure prepared according to Example 1, and showed morphology of irregular spherical particles having diameters of 10-20 nm after characterized by SEM. Isolated Co metal single atoms were distributed on the surface and in the pores of Al₂O₃, and intervals between the metal atoms were 1-10 nm.

1.5 g of Co-Al₂O₃ composite catalyst and 2 ml of ultrapure water were added into a sealable pressure-bearing stainless reaction tube having a volume of 30 ml, 4 bar CO₂ was filled into the tube after air in the tube was replaced with CO₂. The reaction tube was laid flat in an oven, so that the catalyst and water were tiled evenly, and the temperature of the oven was controlled to 135°C±5°C. After reacted for 18 h, an outlet of the reaction tube was connected to GC-FID and GC-TCD to conduct product analysis.

Three Co-Al₂O₃ composite catalysts having loading amounts of 0.25%, 0.5% and 1%, respectively, were evaluated for thermal reaction, concentrations of C₁-C₃ hydrocarbon products in products obtained from stable reactions were about 625.7ppm, 1278.8ppm and 2588.6ppm, respectively, and the calculated catalyst activities per unit corresponding to loading amount of active single atoms of catalysts were about 51.8, 50.7 and 51.4µmol/g/h, respectively.

### Example 7 Photocatalytic reaction for producing hydrocarbon molecules

The photocatalytic reaction was carried out in the same manner as Example 2 by utilizing the Ru-Al₂O₃ composite catalyst prepared according to Example 1. By analysis, a concentration of C₁-C₃ hydrocarbon products in the product obtained from stable reaction was about 927.3 ppm, and a calculated catalyst activity per unit corresponding to active single atom of the catalyst was about 73.60 µmol/g/h.

### Example 8 Photocatalytic reaction for producing hydrocarbon molecules

The photocatalytic reaction was carried out in the same manner as Example 2 by utilizing the RuFe-Al₂O₃ composite catalyst prepared according to Example 1. By analysis, a concentration of C₁-C₃ hydrocarbon products in the product obtained from stable reaction was about 1386.9 ppm, and a calculated catalyst activity per unit corresponding to active single atom of the catalyst was about 110.08 µmol/g/h.

### Example 9 Photocatalytic reaction for producing hydrocarbon molecules

The photocatalytic reaction was carried out in the same manner as Example 2 by using the RuCo-Al₂O₃ composite catalyst prepared according to Example 1. By analysis, a concentration of C₁-C₃ hydrocarbon products in the product obtained from stable reaction was about 1173.6 ppm, and a calculated catalyst activity per unit corresponding to active single atom of the catalyst was about 93.15 µmol/g/h.

### Comparative Examples Plasmonic thermal catalysis conducted by utilizing Co nano particle catalyst

The thermal catalytic reaction was carried out in the same manner as Example 6 by utilizing Co nanostructure catalyst, and the catalyst activity per unit for producing C₁-C₃ hydrocarbon molecules was calculated.

Three Co nano particle catalysts having diameters of 50nm, 100nm and 500nm, respectively, were evaluated for thermal reaction, concentrations of C₁-C₃ hydrocarbon products in products obtained from stable reactions were about 12620 ppm, 9770 ppm and 1104 ppm, respectively, and the calculated catalyst activities per unit corresponding to loading amount of active single atoms of catalysts were about 2.50, 1.94 and 0.22 µmol/g/h, respectively.

As shown in Fig. 4, in terms of the amount of product produced by per unit of effective catalytic metal (Co) in per unit of time (hour), catalyst activities per unit of the plasmonic composite catalyst were stabilized at a very close value and much larger than that of the nano structure catalyst, which indicated that the active single atoms of the single atom catalyst were sufficiently dispersed and exposed, and have extremely high utilization efficiency.

In this specification and the appended claims, the singular forms "a", "an", and "the" include plural reference, unless the context clearly indicates otherwise. Unless defined otherwise, all technical and scientific terms used herein have the same meaning as commonly understood to those skilled in the art. Methods recited herein may be carried out in any order that is logically possible, in addition to a particular order disclosed.

The representative examples are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples and the references to the scientific and patent literature included herein. The examples contain important additional information, exemplification and guidance that can be adapted to the practice of this invention in its various embodiments and equivalents thereof.

## Claims

1. A method for producing hydrocarbon molecules by means of energy radiation, comprising:
contacting a composite catalyst with at least one hydrogen-containing source and at least one carbon-containing source, and
subjecting the composite catalyst, the hydrogen-containing source, and the carbon-containing source to energy radiation, to produce hydrocarbon molecules, wherein
the composite catalyst comprises at least one nano-base structure and at least one atomic sites, the atomic sites comprise one or two or more chemical elements selected from Mn, Co, Fe, Ru, Rh, Al, Ag, Au, Pt, Pd, Cu, Ni, Zn, Ti, Os, Ir and La.

2. The method according to claim 1, wherein
the energy radiation is at least one selected from light radiation and heat radiation, preferably light radiation.

3. The method according to claim 1 or 2, wherein
a distance between the nano-base structure and the atomic site is 5 nm or less, preferably 1 nm or less, more preferably less than 0.1 nm, most preferably the nano-base structure and the atomic site are in close contact with each other.

4. The method according to any of claims 1 to 3, wherein
the atomic sites are bonded with the nano-base structure, for example by physical manner or chemical manner.

5. The method according to any one of claims 1 to 4, wherein
a mass percentage of the atomic site and the nano-base structure is 50% or less, preferably 0.01% to 30%, preferably 0.01% to 5%, more preferably 0.01% to 2%, most preferably 0.1% to 1%.

6. The method according to any one of claims 1 to 5, wherein
the atomic sites are loaded on surface, internal pores of the nano-base structure, or distributed in internal crystal lattices of the nano-base structure, preferably respective atomic sites are dispersed uniformly, and
intervals between respective atomic sites are 0.2-500nm, preferably 1-50nm, more preferably 1-10nm.

7. The method according to any one of claims 1 to 6, wherein
the nano-base structure is selected from a group consisting of Mn, Co, Ce, Fe, Al, Ca, Ce, Cu, Ni, Ti, Zn, Si, Mo, Bi, V, C, N and oxides, nitrides, sulfites, carbides, hydroxides, chlorides thereof and metal-organic frameworks (MOF), preferably metal-organic frameworks, TiO₂ or Al₂O₃.

8. The method according to any one of claims 1 to 7, wherein
the composite catalyst is a catalyst in which Co and Mn are loaded on or bonded to a metal-organic framework, a catalyst in which Fe is loaded on or bonded to Al₂O₃, a catalyst in which Co is loaded on or bonded to Al₂O₃, a catalyst in which Ru is loaded on or bonded to Al₂O₃, a catalyst in which Ru and Fe are loaded on or bonded to Al₂O₃, or a catalyst in which Ru and Co are loaded on or bonded to Al₂O₃.

9. The method according to any one of claims 1 to 8, wherein
the nano-base structure is about 1 nm to about 1000 nm, preferably about 70 nm to about 1000 nm, about 100 nm to about 800 nm, about 200 nm to about 500 nm in at least one dimension of length, width and height.

10. The method according to any one of claims 1 to 9, wherein
the nano-base structure each independently is about 1 nm to about 3000 nm in length, width or height, preferably, about 100 nm to about 3000 nm, about 500 nm to about 2500 nm, or about 1000 nm to about 2000 nm in length, and/or about 1 nm to about 1000 nm, about 70 nm to about 1000 nm, about 100 nm to about 800 nm, or about 200 nm to about 500 nm in width or height, or
the nano-base structure each independently has an aspect ratio of about 1 to about 20, preferably an aspect ratio of about 1 to about 10, or about 2 to about 8.

11. The method according to any one of claims 1 to 10, wherein
the nano-base structure each independently has a shape of spherical, spike, flake, needle, blade of grass, columnar, polyhedral, 3D cone, cuboidal, sheet, hemispherical, irregular 3D shape, porous structure or any combinations thereof.

12. The method according to any one of claims 1 to 11, wherein
a plurality of the atomic sites are arranged in a patterned configuration, preferably in a plurality of layers, on the nano-base structure, or
a plurality of the atomic sites are dispersed randomly in the nano-base structure and/or on an surface of the nano-base structure.

13. The method according to any one of claims 1 to 12, wherein
the energy radiation allows the reaction progresses at a temperature between about 20°C to about 500°C, preferably about 50°C to about 300°C, about 70°C to about 250°C, about 90°C to about 200°C, about 100°C to about 200°C, about 100°C to about 180°C, about 100°C to about 150°C, about 110°C to about 140°C, and about 120°C to about 140°C.

14. The method according to any one of claims 2 to 13, wherein
the reaction is initiated by utilizing light radiation or heat radiation, and the reaction is continued to progress by utilizing light radiation or heat radiation, wherein
a light radiation power of the light radiation is 200-1500 W/m², preferably 200-1000 W/m², most preferably 500-1000 W/m².

15. The method according to any one of claims 2 to 14, wherein
temperatures of the composite catalyst, the hydrogen-containing source and the carbon-containing source are raised by the light radiation, preferably that the light radiation is the sole source for raising the temperatures.

16. The method according to any one of claims 1 to 15, wherein
the carbon-containing source is selected from a group consisting of CO₂, CO, C₁₋₄ hydrocarbons, synthesis gas, bicarbonate salts and any combination thereof, or air, industrial flue gas, exhausts or emissions comprising one or more of these carbon-containing sources, preferably CO₂ or CO.

17. The method according to any one of claims 1 to 16, wherein
the hydrogen-containing source is selected from a group consisting of water, H₂, C₁₋₄ hydrocarbons and any combination thereof, or air, industrial flue gas, exhausts or emissions comprising one or more of these hydrogen-containing sources, preferably water.
